# EUROPÄISCHE PATENTSCHRIFT

(11) **EP 3 647 228 B1**
(45) Veröffentlichungstag und Bekanntmachung des Hinweises auf die Patenterteilung: **22.03.2023**
(21) Anmeldenummer: 18203990.9
(22) Anmeldetag: 01.11.2018
(51) Int. Cl.: A61L 2/06, A01M 13/00, A01M 19/00, F25D 11/00

(54) **VORRICHTUNG ZUR WÄRMEBEHANDLUNG VON KRAFTFAHRZEUGEN ODER KRAFTFAHRZEUGTEILEN**
DEVICE FOR THE HEAT TREATMENT OF MOTOR VEHICLES OR UNITS OF A MOTOR VEHICLE
DISPOSITIF DE TRAITEMENT THERMIQUE DES VÉHICULES AUTOMOBILES OU DES PARTIES DES VÉHICULES AUTOMOBILES

(43) Veröffentlichungstag der Anmeldung: 06.05.2020
(73) Patentinhaber: UNIKAI Lagerei- und Speditionsgesellschaft mbH, 20457 Hamburg (DE)
(72) Erfinder: Albers, Patrick, 20457 Hamburg (DE)
(74) Vertreter: Königer, Karsten

(56) Entgegenhaltungen:
- EP-A1- 1 179 978
- EP-A1- 3 147 601
- EP-A1- 3 586 624
- WO-A1-2007/027111
- DE-A1- 3 208 302
- DE-A1-102015 006 098
- OA-A- 9 942
- US-A- 3 991 589
- US-A- 5 642 827
- US-A1- 2002 170 227
- US-A1- 2008 194 192
- US-A1- 2011 209 321
- US-A1- 2013 052 595

## Beschreibung

Gegenstand der Erfindung ist eine Verwendung einer Vorrichtung zur Wärmebehandlung von Kraftfahrzeugen oder Kraftfahrzeugteilen, insbesondere zum Zwecke der Abtötung von Schädlingen, sowie eine Vorrichtung zur Wärmebehandlung von Kraftfahrzeugen oder Kraftfahrzeugteilen.

Unter Wärmebehandlung oder Hitzebehandlung von Kraftfahrzeugen wird verstanden, ein Kraftfahrzeug in der Weise zu erwärmen, dass das Kraftfahrzeug an seiner kältesten Stelle für eine bestimmte Zeit eine bestimmte Mindesttemperatur übersteigt. Eine solche Wärmebehandlung kann insbesondere dazu dienen, gesetzliche oder behördlichen Bestimmungen zu erfüllen, die eine Behandlung von zu exportierenden oder zu importierenden Gütern zur Schädlingsbekämpfung verlangen. Insbesondere verlangen zurzeit Australien und Neuseeland zur Bekämpfung der braun marmorierten Baumwanze (brown marmorated stink bug, BMSB), dass Kraftfahrzeuge, die innerhalb eines bestimmten Zeitraums des Jahres aus bestimmten Ländern importiert werden, innerhalb von 120 Stunden vor Abfahrt beispielsweise des Schiffes behandelt werden. Als ausreichend anerkannt ist dabei zurzeit, jedes Kraftfahrzeug so zu erhitzen, dass er an seiner kältesten Stelle für eine Zeit von mindestens 20 Minuten eine Temperatur von mindestens 50 Grad Celsius (Australien) bzw. für eine Zeit von mindestens 30 Minuten eine Temperatur von mindestens 56 Grad Celsius (Neuseeland) hat. Dabei muss die Behandlung für jedes Kraftfahrzeug individuell mit allen Parametern dokumentiert werden. Aufgrund der Schiffsgrößen sind für eine Schiffsladung bis zum 2.000 Fahrzeuge einer solchen Behandlung zu unterziehen.

Bekannt ist es, eine solche Wärmebehandlung in der Weise durchzuführen, dass ein Lagerhaus dafür ertüchtigt wird oder für diesen Zweck errichtet wird. Diese Lösung geht jedoch mit hohen Investitionen und langen baurechtlichen Genehmigungszeiten einher.

Aus EP 1 179 978 A1 / WO 00/62607 ist eine Begasungsvorrichtung bekannt, die einen Schiffscontainer umfasst, der eine Begasungskammer definiert, die daran angepasst ist, zu begasende Erzeugnisse zu enthalten. Aus WO 2007/027111 A1 ist die Verwendung eines Seefracht-Containers bekannt, an den für eine Wärmebehandlung eine externe Heizeinrichtung angeschlossen ist.

Aufgabe der Erfindung ist es, Lösungen zur Wärmebehandlung von Kraftfahrzeugen oder Kraftfahrzeugteilen, insbesondere zum Zwecke der Abtötung von Schädlingen, zur Verfügung zu stellen, die gegenüber bekannten Lösungen Vorteile aufweisen.

Diese Aufgabe wird gelöst durch eine Verwendung einer Vorrichtung gemäß Anspruch 1 sowie eine Vorrichtung gemäß Anspruch 5. Besonders vorteilhafte Ausführungsformen der Erfindung sind Gegenstand der Unteransprüche.

Ein isolierter Seefracht-Container für einen temperaturgeführten Transport, auch als Kühlcontainer bezeichnet, ist ein gegenüber Standard-Seefracht-Containern besonders wärmeisolierter Großraumbehälter, der typischerweise für temperaturempfindliche Fracht verwendet wird. Insbesondere gibt es Kühlcontainer mit integrierter Kälteanlage (Kühleinheit), wobei die Kühleinheit mit elektrischem Strom betrieben wird. Solche Kühlcontainer werden auch als Integralcontainer bezeichnet. Kühlcontainer haben eine im Wesentlichen rechteckige Grundform mit einer langen Achse von typischerweise 40 Fuß (ca. 12 m) Länge. Die Ladeöffnung des Containers ist typischerweise an einer der beiden Stirnseiten, während die Kühleinheit an der anderen Stirnseite angeordnet ist, typischerweise durch eine Aufnahmeöffnung eingeschoben. Dabei kann die Kühleinheit über einen umlaufenden Flansch mit Bolzenverbindungen rings um den Außenrand der Aufnahmeöffnung lösbar befestigt werden. In der Kühleinheit gekühlte Luft kann in den Containerinnerraum strömen. Der Innenboden eines Kühlcontainers kann mit Längsrippen so ausgebildet sein, dass zwischen den Rippen Längskanäle für Kühlluft bleiben. Typischerweise wird so eine Luftzirkulation innerhalb des Containers ermöglicht.

Für die Funktionsfähigkeit der Erfindung ist es nicht erforderlich, dass der Container ein Kühlaggregat aufweist oder dass das Kühlaggregat funktionsfähig ist.

Die Verwendung eines Kühlcontainers hat den Vorteil, dass gebrauchte Kühlcontainer günstig zu erwerben sind. Dies gilt erst recht, wenn das Kühlaggregat defekt ist.

Durch die ohnehin vorhandene Wärmeisolierung ergibt sich im Vergleich zu einem Lagerhaus eine hohe Energieeffizienz pro behandeltem Fahrzeug.

Im Vergleich zu einem zur Wärmebehandlung ertüchtigten Lagerhaus ist bei der erfindungsgemäßen Lösung die Installation der Messfühler deutlich weniger aufwendig. Auch ist die Installation der Lüftungstechnik zur Erhitzung des Innenraums des Kraftfahrzeugs deutlich weniger aufwendig. Insbesondere die Installation einer Unterbodenlüftungstechnik ist bei der erfindungsgemäßen Lösung deutlich weniger aufwendig.

Eine Anlage, die aus mehreren Kühlcontainern besteht, hat gegenüber einer zur Wärmebehandlung ertüchtigten Lagerhalle, die typischerweise deutlich größer als ein Kühlcontainer ist, den weiteren Vorteil, dass dadurch, dass in jedem Kühlcontainer nur eine kleine Anzahl von Kraftfahrzeugen, typischerweise zwei, wärmebehandelt wird, eine individuelle Wärmebehandlung einer kleinen Anzahl von Kraftfahrzeugen im Hinblick auf Mindesttemperatur und Mindestdauer problemlos möglich ist.

Die Maximalkapazität einer Anlage, die aus mehreren Kühlcontainern besteht, ist frei skalierbar. Während der benutzungsfreien Zeit lassen sich die Container effizient lagern bzw. stapeln, auch außerhalb des Hafens, wodurch eine nutzlose Belegung von teuren Hafenflächen vermieden wird. Da die Umrüstung eines Kühlcontainers zu einer erfindungsgemäßen Vorrichtung nur einen geringen Eingriff darstellt, können die Container auch zurückgerüstet werden und wieder für ihren ursprünglichen Zweck verwendet werden.

Die vorgeschlagenen mobilen Warmwasserquellen können gemietet und genehmigungsfrei aufgestellt werden. Dadurch entfallen Kapitalkosten für die nutzungsfreie Zeit.

Vorzugsweise sind der mindestens eine Ventilator und der mindestens eine Wärmetauscher in dem Container angeordnet. Dadurch werden Energieverluste durch eine Anordnung außerhalb des Containers vermieden.

Vorzugsweise sind der mindestens eine Ventilator und der mindestens eine Wärmetauscher in einer Einheit integriert. Solche Einheiten werden fertig auf dem Markt angeboten.

Vorzugsweise umfasst der Container eine Kühleinheit, die mit elektrischem Strom betrieben werden kann und die ein Kühlaggregat, das möglicherweise funktionsfähig ist, und mindestens einen Ventilator umfasst. Der ohnehin bereits vorhandene Ventilator der Kühleinheit kann dann ebenfalls zur Bewegung und Verteilung der Luft genutzt werden. Dabei ist die Kühleinheit vorzugsweise an einer der beiden Stirnseiten des Containers angeordnet. Vorzugsweise bildet die Kühleinheit einen vertikalen Schacht an der Innenseite der Stirnseite, wobei die Rückwand der Kühleinheit funktionell die Wand des Containers ergänzt oder mit dieser identisch ist, wobei die der Rückwand der Kühleinheit gegenüberliegende Querwand mindestens eine Durchtrittsöffnung aufweist, durch die Luft aus dem Schacht in den Container und/oder aus dem Container in den Schacht strömen kann.

Vorzugsweise wird bei der erfindungsgemäßen Verwendung einer der Stoffe, die durch den Wärmetauscher strömen, durch eine Leitung von außerhalb des Containers zugeführt und durch eine Leitung nach außerhalb des Containers abgeführt. Bei der erfindungsgemäßen Vorrichtung ist dies der Fall.

Vorzugsweise handelt es sich bei den Kraftfahrzeugen um Personenkraftwagen.

Vorzugsweise ist der mindestens eine Wärmetauscher ein Wasser-Luft-Wärmetauscher. Vorzugsweise wird der Wärmetauscher von einer mobilen Warmwasserquelle versorgt. Vorzugsweise beträgt die Wärmeleistung der Wärmetauscher insgesamt mindestens 100 kW. Vorzugsweise weist der Container an jeder der beiden Stirnseiten mindestens einen Wärmetauscher und einen Ventilator auf. Vorzugsweise umfasst die Vorrichtung zwei Wärmetauscher, deren Wärmeleistung jeweils mindestens 50 kW beträgt.

Vorzugsweise ist eine Inspektionsklappe in der Querwand der Kühleinheit durch ein Blech ersetzt, das jeweils einen Vorlauf- und einen Rücklaufschlauch pro Wärmetauscher durchlässt.

Vorzugsweise kann mindestens ein Rohr parallel zur Längsseite des Containers mit mindestens zwei Ausgängen Luft im Containerinnenraum verteilen. Besonders vorteilhaft ist es, wenn auf das Rohr ein Radialventilator aufgesteckt ist. Dabei sind die Ausgänge vorzugweise regulierbar. Vorzugsweise sind die Ausgänge in regelmäßigen Abständen angeordnet. Vorzugsweise ist das mindestens eine Rohr ein Wickelfalzrohr. Vorzugsweise ist jeweils ein Rohr im Bereich der Decke jeweils im Bereich einer Längsseitenwand angeordnet. Durch diese Maßnahmen ist es möglich, die von einem Wärmetauscher erwärmte Luft gezielt auch in Bereiche des zu erwärmenden Kraftfahrzeugs zu leiten, deren Erwärmung sonst deutlich länger dauern würde.

Vorzugsweise sind am Boden des Containers angeordnete Rippen, zwischen denen Luft strömen kann, teilweise abgedeckt, so dass zwischen den Rippen strömende Luft nur durch bestimmte Öffnungen nach oben strömen kann. Vorzugsweise wird dabei Luft durch mindestens einen in der Kühleinheit angeordneten Ventilator in die Räume zwischen den Rippen gedrückt wird. Vorzugsweise sind die Öffnungen regulierbar. Vorzugsweise sind die Öffnungen in regelmäßigen Abständen angeordnet. Auch diese Maßnahmen ermöglichen eine gezielte Leitung erwärmter Luft in Bereiche des zu erwärmenden Kraftfahrzeugs, deren Erwärmung sonst deutlich länger dauern würde. Durch die Nutzung von im Boden des Kühlcontainers ohnehin vorhandenen Rippen und die Nutzung eines Ventilators der Kühleinheit ist die Installation der Unterbodenlüftungstechnik deutlich einfacher als bei der Ertüchtigung einer Lagerhalle zur Wärmebehandlung.

Vorzugsweise ist eine Beleuchtungsvorrichtung im Containerinnenraum angeordnet. Vorzugsweise ist ein Not-aus-Schalter vorhanden.

Vorzugsweise weist die Vorrichtung an der Ladeöffnung klappbare Auffahrrampen auf. Vorzugsweise sind die Laderampen (Auffahrrampen) mit dem Container lösbar verbindbar. Vorzugsweise können die Laderampen über mindestens eine Klemmvorrichtung an Kühlrippen am Boden des Containers lösbar befestigt werden.

Vorzugsweisedurch können durch Kombination mehrerer Container in der Länge, Breite und/oder Höhe größere zu behandelnde Frachtstücke oder Kraftfahrzeuge erhitzt werden.

Vorzugsweise ist im Containerinnenraum mindestens ein drahtgebundener Messfühler angeordnet. Vorzugsweise können von dem Messfühler gemessene oder aufgrund der gemessenen Werte errechnete Werte an ein Gerät übermittelt werden, das zur Dokumentation der Werte geeignet ist.

Vorzugsweise wird, wenn alle Messwerte einen vorgegebenen Grenzwert erreicht haben, die Zeit gemessen, während der sämtliche Werte ununterbrochen in dem vorgegebenen Bereich bleiben, und wird nach Ablauf einer vorgegebenen Mindestzeit ein optisch oder akustisch wahrnehmbares Signal erzeugt.

Vorzugsweise beträgt die Vorlauftemperatur für den mindestens einen Wärmetauscher maximal 95 Grad Celsius.

Vorzugsweise liegt ein System vor, das vier erfindungsgemäße Vorrichtungen umfasst, wobei vier Container durch eine gemeinsame 400 kW-Warmwasserquelle versorgt werden.

Die Erfindung wird anhand der Figuren näher erläutert. Es zeigen
- Figur 1:: eine erfindungsgemäße Vorrichtung
- Figur 2:: einen Grundriss einer erfindungsgemäßen Vorrichtung
- Figur 3:: eine Rückwand einer erfindungsgemäßen Vorrichtung von außen

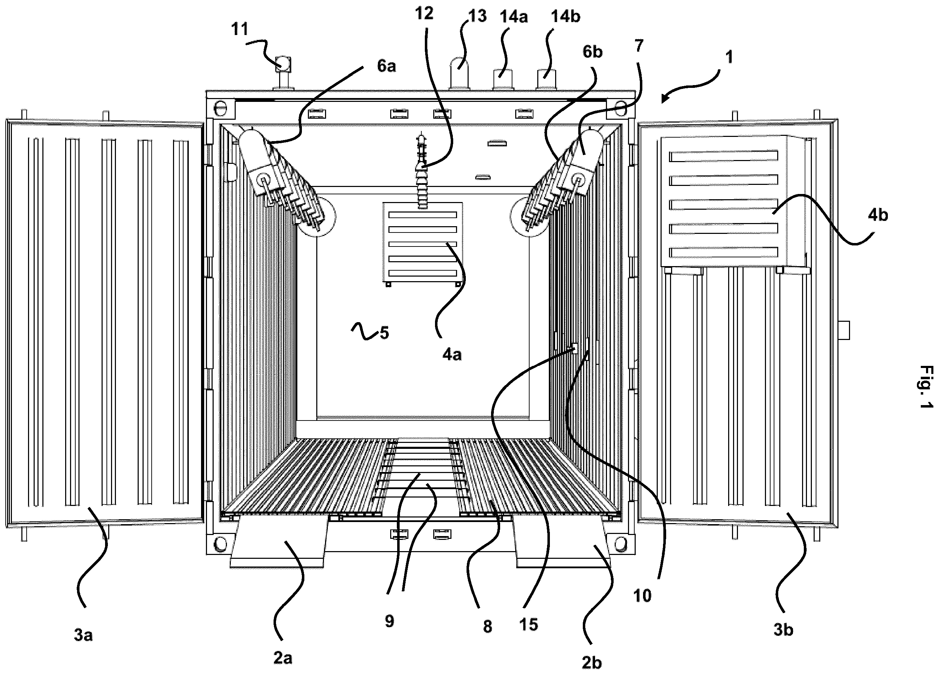

Figur 1 zeigt eine erfindungsgemäße Vorrichtung in einem Zustand mit geöffneter Ladeöffnung des isolierten Seefracht-Containers 1 und ausgeklappten Laderampen 2a und 2b. Ein Kraftfahrzeug, das beispielsweise zum Zwecke der Abtötung von Schädlingen wärmebehandelt werden soll, kann über die Laderampen 2a, 2b in den Containerinnenraum rollen. Die Ladeöffnung kann durch Verschwenken der beiden Flügeltüren 3a und 3b geschlossen werden, nachdem die Laderampen 2a und 2b hochgeklappt worden sind. Wenn die Ladeöffnung geschlossen ist, ist an den beiden Stirnseiten des Innenraums des Containers 1 jeweils eine Einheit 4a, 4b angeordnet, in der jeweils ein Wärmetauscher und ein Ventilator integriert sind. An der der Ladeöffnung gegenüberliegenden Stirnseite des Innenraums des Containers 1 befindet sich eine Querwand 5, hinter der sich eine nicht gezeigte Kühleinheit, die zwei Ventilatoren umfasst, befindet. Die in der Figur nicht gezeigte Kühleinheit bildet einen vertikalen Schacht. Die Rückwand der Kühleinheit ergänzt funktionell die der Ladeöffnung gegenüberliegende Wand des Containers. Die Querwand 5 weist nicht gezeigte Durchtrittsöffnungen auf, durch die Luft aus dem Schacht in den Containerinnenraum strömen kann. Im Bereich der Decke des Containerinnenraums ist jeweils im Bereich einer Längsseitenwand ein Rohr 6a, 6b parallel zur Längsseite angeordnet, das über mehrere regulierbare Ausgänge 7, die in regelmäßigen Abständen angeordnet sind, Luft im Containerinnenraum verteilen kann. An dem der Ladeöffnung abgewandten Ende des Rohres ist jeweils ein nicht gezeigter Radialventilator aufgesteckt. Am Boden des Containers sind Rippen 8 angeordnet, durch die Luft strömen kann, die durch den nicht gezeigten Ventilator in der Kühleinheit in die Räume zwischen den Rippen gedrückt wird. Die Rippen sind durch Platten 9 teilweise abgedeckt, so dass zwischen den Rippen strömende Luft nur durch die zwischen den Platten vorhandenen Öffnungen nach oben strömen kann. An eine Längswand des Containerinnenraums hängen an Kabeln drahtgebundene Messfühler 10. Gezeigt sind ferner eine Hupe 11, eine Sprinkleranlage 12, eine Rundumleuchte 13, zwei Betriebsanzeigen 14a, 14b und ein Not-Aus-Schalter 15.

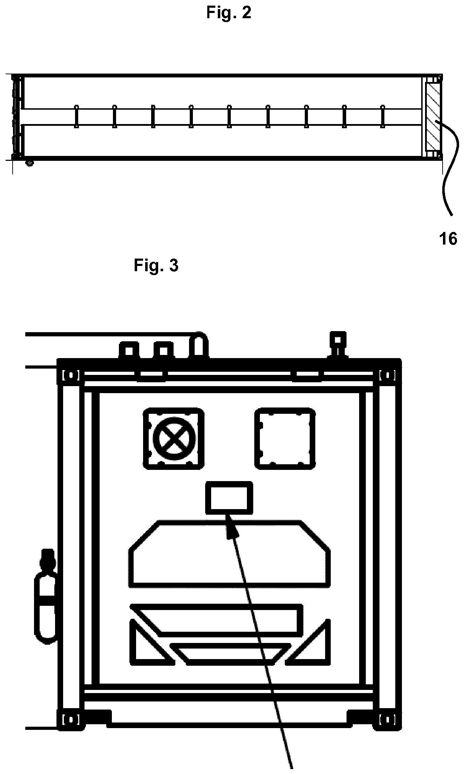

Der in Figur 2 gezeigte Grundriss zeigt die Anordnung der Kühleinheit 16 innerhalb des Kühlcontainers.

Figur 3 zeigt schematisch die der Ladeöffnung gegenüberliegen Wand des Containers von außen. Diese Rückwand wird funktionell ergänzt durch die Rückwand der Kühleinheit.

## Patentansprüche

1. Verwendung einer Vorrichtung zur Wärmebehandlung von Kraftfahrzeugen oder Kraftfahrzeugteilen, insbesondere zum Zwecke der Abtötung von Schädlingen, wobei die Vorrichtung einen isolierten Seefracht-Container für einen temperaturgeführten Transport (1), mindestens einen Ventilator und zusätzlich zu einem möglicherweise im Container (1) angeordneten Kühlaggregat, das möglicherweise funktionsfähig ist, mindestens einen Wärmetauscher umfasst.

2. Verwendung nach Anspruch 1, **dadurch gekennzeichnet, dass** der mindestens eine Ventilator und der mindestens eine Wärmetauscher in dem Container (1) angeordnet sind.

3. Verwendung nach einem der vorhergehenden Ansprüche, **dadurch gekennzeichnet, dass** der mindestens eine Ventilator und der mindestens eine Wärmetauscher in einer Einheit (4a, 4b) integriert sind.

4. Verwendung nach einem der vorhergehenden Ansprüche, **dadurch gekennzeichnet, dass** im Containerinnenraum mindestens ein drahtgebundener Messfühler (10) angeordnet ist und dass, wenn alle Messwerte einen vorgegebenen Grenzwert erreicht haben, die Zeit gemessen wird, während der sämtliche Werte ununterbrochen in dem vorgegebenen Bereich bleiben und nach Ablauf einer vorgegebenen Mindestzeit ein optisch oder akustisch wahrnehmbares Signal erzeugt ist.

5. Vorrichtung zur Wärmebehandlung von Kraftfahrzeugen oder Kraftfahrzeugteilen, insbesondere zum Zwecke der Abtötung von Schädlingen, umfassend einen isolierten Seefracht-Container für einen temperaturgeführten Transport (1), wobei die Vorrichtung mindestens einen Ventilator und zusätzlich zu einem möglicherweise im Container angeordneten Kühlaggregat, das möglicherweise funktionsfähig ist, mindestens einen Wärmetauscher umfasst, **dadurch gekennzeichnet, dass** einer der Stoffe, die durch den Wärmetauscher strömen, durch eine Leitung von außerhalb des Containers zugeführt und durch eine Leitung nach außerhalb des Containers abgeführt wird.

6. Vorrichtung nach Anspruch 5, **dadurch gekennzeichnet, dass** der Wärmetauscher von einer mobilen Warmwasserquelle versorgt wird.

7. Vorrichtung nach einem der Ansprüche 5 bis 6, **dadurch gekennzeichnet, dass** der Container (1) an jeder der beiden Stirnseiten mindestens einen Wärmetauscher und einen Ventilator aufweist, wobei die Wärmeleistung der beiden Wärmetauscher jeweils mindestens 50 kW beträgt.

8. Vorrichtung nach einem der Ansprüche 5 bis 7, **dadurch gekennzeichnet, dass** mindestens ein Rohr (6a, 6b) parallel zur Längsseite des Containers (1) mit mindestens zwei Ausgängen (7) Luft im Containerinnenraum verteilen kann.

9. Vorrichtung nach einem der Ansprüche 5 bis 8, **dadurch gekennzeichnet, dass** jeweils ein Rohr (6a, 6b) im Bereich der Decke jeweils im Bereich einer Längsseitenwand angeordnet ist, wobei auf das Rohr (6a, 6b) ein Radialventilator aufgesteckt ist.

10. Vorrichtung nach einem der Ansprüche 5 bis 9, **dadurch gekennzeichnet, dass** der Container (1) eine Kühleinheit (16) umfasst, die mit elektrischen Strom betrieben werden kann und die ein Kühlaggregat, das möglicherweise funktionsfähig ist, und mindestens einen Ventilator umfasst, wobei die Kühleinheit an einer der beiden Stirnseiten des Containers (1) angeordnet ist, wobei die Kühleinheit (16) einen vertikalen Schacht an der Innenseite der Stirnseite bildet, wobei die Rückwand der Kühleinheit (16) funktionell die Wand des Containers (1) ergänzt oder mit dieser identisch ist, wobei die der Rückwand der Kühleinheit (16) gegenüberliegende Querwand (5) mindestens eine Durchtrittsöffnung aufweist, durch die Luft aus dem Schacht in den Container (1) und/oder aus dem Container (1) in den Schacht strömen kann, wobei am Boden des Containers (1) angeordnete Rippen (8), zwischen denen Luft strömen kann, teilweise abgedeckt sind, so dass zwischen den Rippen (8) strömende Luft nur durch bestimmte Öffnungen nach oben strömen kann, wobei Luft durch mindestens einen in der Kühleinheit (16) angeordneten Ventilator in die Räume zwischen den Rippen (8) gedrückt wird.

11. Vorrichtung nach einem der Ansprüche 5 bis 10, **dadurch gekennzeichnet, dass** sie an einer Ladeöffnung klappbare Laderampen (2a, 2b) aufweist, wobei die Laderampen (2a, 2b) über mindestens eine Klemmvorrichtung an Kühlrippen am Boden des Containers lösbar befestigt werden können.

12. Vorrichtung nach einem der Ansprüche 4 bis 10, **dadurch gekennzeichnet, dass** durch Kombination mehrerer Container (1) in der Länge, Breite und/oder Höhe größere zu behandelnde Frachtstücke oder Kraftfahrzeuge erhitzt werden können.

13. System umfassend vier Vorrichtungen jeweils nach einem der Ansprüche 5 bis 12, **dadurch gekennzeichnet, dass** vier Container (1) durch eine gemeinsame 400 kW-Warmwasserquelle versorgt werden.

## Claims

1. Use of an apparatus for heat treatment of motor vehicles or motor vehicle parts, in particular for the purpose of killing pests, wherein the apparatus comprises an insulated sea freight container (1) for temperature-controlled transportation, at least one fan, a refrigeration unit which is optionally arranged within the container (1) and which is optionally functional, and at least one heat exchanger.

2. Use according to claim 1, **characterized in that** the at least one fan and the at least one heat exchanger are arranged within the container (1).

3. Use according to any one of the preceding claims, **characterized in that** the at least one fan and the at least one heat exchanger are integrated in one unit (4a, 4b).

4. Use according to any one of the preceding claims, **characterized in that** at least one wired measuring sensor (10) is arranged in the container interior, and **in that**, when all measured values have reached a predetermined limit value, the time during which all the values remain continuously in the predetermined range is measured, and an optically or acoustically perceptible signal is produced after the expiry of a predetermined minimum time.

5. Apparatus for heat treatment of motor vehicles or motor vehicle parts, in particular for the purpose of killing pests, comprising an insulated sea freight container (1) for temperature-controlled transportation, wherein the apparatus comprises at least one fan, a refrigeration unit which is optionally arranged within the container and which is optionally functional, and at least one heat exchanger, **characterized in that** one of the substances flowing through the heat exchanger is fed in through a line from outside the container and is discharged through a line to the outside of the container.

6. Apparatus according to claim 5, **characterized in that** the heat exchanger is supplied by a mobile hot water source.

7. Apparatus according to any one of claims 5 to 6, **characterized in that** the container (1) has at least one heat exchanger and one fan at each of the two ends, wherein the heat output of each of the two heat exchangers is at least 50 kW.

8. Apparatus according to any one of claims 5 to 7, **characterized in that** at least one pipe (6a, 6b) parallel to the longitudinal side of the container (1), having at least two outlets (7), can distribute air in the container interior.

9. Apparatus according to any one of claims 5 to 8, **characterized in that** a respective pipe (6a, 6b) is arranged in the region of the roof in the region of each longitudinal side wall, wherein a radial fan is mounted on the pipe (6a, 6b).

10. Apparatus according to any one of claims 5 to 9, **characterized in that** the container (1) comprises a cooling unit (16) which can be operated by electric current and which comprises a refrigeration unit that is optionally functional, and at least one fan, wherein the cooling unit is arranged on one of two ends of the container (1), wherein the cooling unit (16) forms a vertical shaft on the inside of the end, wherein the rear wall of the cooling unit (16) functionally complements the wall of the container (1) or is identical therewith, wherein the transverse wall (5) situated opposite the rear wall of the cooling unit (16) has at least one through opening, through which air can flow out of the shaft into the container (1) and/or out of the container (1) into the shaft, wherein ribs (8), which are arranged on the floor of the container (1) and between which air can flow, are partially covered so that air flowing between the ribs (8) can flow upward only through particular openings, wherein air is forced into spaces between the ribs (8) by at least one fan arranged in the cooling unit (16).

11. Apparatus according to any one of claims 5 to 10, **characterized in that** it comprises hinged loading ramps (2a, 2b) at a loading opening, wherein the loading ramps (2a, 2b) can be detachably secured by at least one clamping device to cooling ribs on the floor of the container.

12. Apparatus according to any one of claims 4 to 10, **characterized in that** relatively large items of freight or motor vehicles that are to be treated can be heated by combining a plurality of containers (1) lengthwise, transversely and/or vertically.

13. System comprising four apparatuses, each according to any one of claims 5 to 12, **characterized in that** four containers (1) are supplied by a common 400 kW hot water source.

## Revendications

1. Utilisation d'un dispositif de traitement thermique de véhicules automobiles ou de parties de véhicules automobiles, notamment à des fins d'élimination d'organismes nuisibles, le dispositif comprenant un conteneur maritime isolé pour le transport à température contrôlée (1), au moins un ventilateur et, en plus d'un groupe frigorifique disposé le cas échéant dans le conteneur (1) et éventuellement en état de fonctionnement, au moins un échangeur de chaleur.

2. Utilisation selon la revendication 1, **caractérisée en ce que** le ventilateur, au nombre d'au moins un, et l'échangeur de chaleur, au nombre d'au moins un, sont installés dans le conteneur (1).

3. Utilisation selon l'une des revendications précédentes, **caractérisée en ce que** le ventilateur, au nombre d'au moins un, et l'échangeur de chaleur, au nombre d'au moins un, sont intégrés dans une unité (4a, 4b).

4. Utilisation selon l'une des revendications précédentes, **caractérisée en ce qu'**au moins un capteur de mesure (10) filaire est disposé à l'intérieur du conteneur, et **en ce que**, lorsque toutes les valeurs mesurées ont atteint une valeur limite prédéterminée, on mesure le temps pendant lequel l'ensemble des valeurs reste de manière ininterrompue dans la plage prédéfinie, et après écoulement d'un temps minimum prédéterminé, un signal est généré qui est perceptible sur le plan optique ou acoustique.

5. Dispositif de traitement thermique de véhicules automobiles ou de parties de véhicules automobiles, notamment à des fins d'élimination d'organismes nuisibles, comprenant un conteneur maritime isolé pour le transport à température contrôlée (1), le dispositif comprenant au moins un ventilateur et, en plus d'un groupe frigorifique disposé le cas échéant dans le conteneur (2) et éventuellement en état de fonctionnement, au moins un échangeur de chaleur, **caractérisé en ce que** l'une des substances qui s'écoulent à travers l'échangeur de chaleur est amenée par une conduite depuis l'extérieur du conteneur et est évacuée par une conduite vers l'extérieur du conteneur.

6. Dispositif selon la revendication 5, **caractérisé en ce que** l'échangeur de chaleur est alimenté par une source d'eau chaude mobile.

7. Dispositif selon l'une des revendications 5 à 6, **caractérisé en ce que** le conteneur (1) présente, sur chacune des deux faces frontales, au moins un échangeur de chaleur et un ventilateur, la puissance calorifique des deux échangeurs de chaleur étant respectivement d'au moins 50 kW.

8. Dispositif selon l'une des revendications 5 à 7, **caractérisé en ce qu'**au moins un tube (6a, 6b) peut répartir de l'air à l'intérieur du conteneur avec au moins deux sorties (7), parallèlement au grand côté du conteneur (1).

9. Dispositif selon l'une des revendications 5 à 8, **caractérisé en ce qu'**un tube (6a, 6b) est disposé respectivement dans la région du plafond, chaque fois dans la région d'une paroi latérale longitudinale, un ventilateur radial étant rapporté sur le tube (6a, 6b).

10. Dispositif selon l'une des revendications 5 à 9, **caractérisé en ce que** le conteneur (1) comprend une unité de refroidissement (16) qui peut fonctionner avec du courant électrique et comprend un groupe frigorifique, éventuellement en état de fonctionnement, et au moins un ventilateur, l'unité de refroidissement étant disposée sur l'une des faces frontales du conteneur (1), l'unité de refroidissement (16) formant un conduit vertical sur la face intérieure de la face frontale, la paroi arrière de l'unité de refroidissement (16) complétant sur le plan fonctionnel la paroi du conteneur (1) ou est identique à celle-ci, la paroi transversale (5), qui est située en vis-à-vis de la paroi arrière de l'unité de refroidissement (16), présentant au moins une ouverture de passage à travers laquelle de l'air peut s'écouler depuis le conduit dans le conteneur (1) et/ou depuis le conteneur (1) dans le conduit, des nervures (8), qui sont disposées sur le plancher du conteneur (1) et entre lesquelles l'air peut s'écouler, étant en partie recouvertes, de sorte que l'air s'écoulant entre les nervures (8) ne peut s'écouler vers le haut qu'à travers certaines ouvertures, l'air étant poussé dans les espaces entre les nervures (8) par au moins un ventilateur installé dans l'unité de refroidissement (16).

11. Dispositif selon l'une des revendications 5 à 10, **caractérisé en ce qu'**il présente des rampes de chargement (2a, 2b) rabattables sur une ouverture de chargement, les rampes de chargement (2a, 2b) pouvant être fixées de manière amovible par l'intermédiaire d'au moins un dispositif de serrage à des nervures de refroidissement sur le plancher du conteneur.

12. Dispositif selon l'une des revendications 4 à 10, **caractérisé en ce que** la combinaison de plusieurs conteneurs (1) dans le sens de la longueur, de la largeur et/ou de la hauteur permet de chauffer des éléments de fret ou des véhicules de grandes dimensions devant être traités.

13. Système comprenant quatre dispositifs, respectivement selon l'une des revendications 5 à 12, **caractérisé en ce que** quatre conteneurs (1) sont alimentés par une source d'eau chaude commune de 400 kW.
